# EUROPÄISCHE PATENTSCHRIFT

(11) **EP 0 544 880 B1**
(45) Veröffentlichungstag und Bekanntmachung des Hinweises auf die Patenterteilung: **13.03.1996**
(21) Anmeldenummer: 92913299.1
(22) Anmeldetag: 24.06.1992
(51) Int. Cl.: C07C 69/95, A61K 31/235

(54) **VERFAHREN ZUR HERSTELLUNG VON DIACETYLRHEIN**
METHOD OF PREPARING DIACETYL RHEIN
PROCEDE DE PREPARATION DE DIACETYLRHEINE

(30) Priorität: 25.06.1991 DE 4120990
(43) Veröffentlichungstag der Anmeldung: 09.06.1993
(73) Patentinhaber: MADAUS Aktiengesellschaft, D-51075 Köln (DE)
(72) Erfinder: CARCASONA, Alfons, D-5000 Köln 80 (DE); GRIMMINGER, Wolf, D-5060 Bergisch-Gladbach 1 (DE); HIETALA, Pentti, SF-00660 Helsinki 66 (FI); ZAESKE, Helga, D-5063 Overath 3 (DE); WITTHOHN, Klaus, D-5063 Overath 3 (DE)
(86) Internationale Anmeldenummer: EP9201427
(87) Internationale Veröffentlichungsnummer: WO9300322

(56) Entgegenhaltungen:
- DE-A- 3 200 131
- FR-A- 2 508 798
- GB-A- 2 112 640
- TETRAHEDRON, Bd. 23, Nr. 1, Januar 1967, OXFORD GB Seiten 515 - 518; V.K.MURTY ET AL: 'Chemical examination of Cassia fistula'

## Beschreibung

Die Erfindung betrifft ein Verfahren zur Herstellung von Diacetylrhein in pharmazeutisch brauchbarer Reinheit mit einem Restgehalt an unerwünschten Aloeemodinderivaten von insgesamt weniger als 20 ppm, das nach diesem Verfahren erhältliche Diacetylrhein und ein pharmazeutisches Mittel, das diese Verbindung enthält.

Diacetylrhein der Formel: 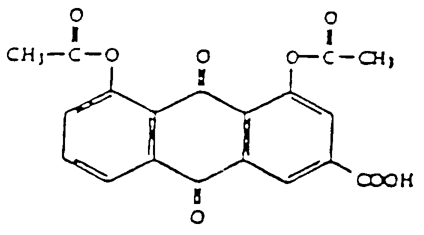 ist ein Arzneiwirkstoff, der antiarthritische, antiinflammatorische, antipyretische und analgetische Aktivität besitzt. Diacetylrhein wird daher zur Behandlung von Arthritiserkrankungen eingesetzt, siehe beispielsweise DE-A 27 11 493 und US-A- 4 244 968.

Diacetylrhein läßt sich beispielsweise durch Acetylierung von Barbaloin und Oxidation des erhaltenen peracetylierten Barbaloins mit Chromtrioxid herstellen. Außerdem läßt sich Diacetylrhein durch Acetylierung von Rhein herstellen, das beispielsweise aus der Sennadroge gewonnen werden kann (siehe DRUGS OF THE FUTURE Vol. IV, No 6, 1979, Seiten 445-447).

In dem nach diesem Verfahren erhaltenen Diacetylrhein sind als unerwünschte Begleitstoffe Aloeemodinderivate enthalten, die von einer unvollständigen Oxidation mit Chromtrioxid herrühren oder bei der Extraktion der Sennadroge mitextrahiert werden. Diese Begleitstoffe sind in relativ geringen Mengen enthalten und lassen sich daher anhand klassischer Reinigungsoperationen nur sehr schwierig abtrennen. Außerdem fallen bei dem oben zuerst genannten Verfahren Chromrückstände an, die in geeigneter Weise entsorgt werden müssen.

Der vorliegenden Erfindung liegt daher die Aufgabe zugrunde, ein Verfahren zur Gewinnung von Diacetylrhein zur Verfügung zu stellen, das einfach und mit hoher Ausbeute durchzuführen ist, bei dem das Diacetylrhein in pharmazeutisch brauchbarer Reinheit mit einem Restgehalt an unerwünschten Aloeemodinderivaten von insgesamt weniger als 20 ppm anfällt.

Diese Aufgabe wird gelöst durch das erfindungsgemäße Verfahren, das dadurch gekennzeichnet ist, daß man
- A): Aloeemodinkomponenten (d.h. Aloeemodin und/oder Derivate davon) enthaltendes Rhein-9-anthron-8-glucosid einer Flüssig-Flüssig-Verteilung zwischen einem nur teilweise mit Wasser mischbaren polaren organischen Lösungsmittel und einer wäßrigen Phase unterwirft,
- B): das nach der Verteilung in der wäßrigen Phase enthaltende Rhein-9-anthron-8-glucosid zu Rhein-8-glucosid oxidiert,
- C): den Glucoserest in 8-Stellung des Rhein-8-glucosids in saurem Medium abspaltet und
- D): das erhaltene Rhein acetyliert und das Diacetylrhein gewinnt

Eine wesentliche Quelle für das Rhein-9-anthron-8-glucosid sind die in der Sennadroge enthaltenen Sennoside. Eine bevorzugte Ausführungsform der Erfindung ist daher ein Verfahren zur Herstellung von Diacetylrhein, das im wesentlichen frei von Aloeemodinderivaten ist, wobei man
- A): ein Sennosidgemisch einer Reduktion zu den entsprechenden Rhein-9-anthron-8-glucosid und Aloe-emodin-9-anthron-8-glucosid-Verbindungen unterwirft,
- B): eine Flüssig-Flüssig-Verteilung der erhaltenen Verbindungen zwischen einem nur teilweise mit Wasser mischbaren, polaren organischen Lösungsmittel und einer wäßrigen Phase durchführt,
- C): die nach der Verteilung in der wäßrigen Phase enthaltenen Rhein-9-anthron-8-glucosid-Verbindungen zu der entsprechenden Anthrachinonverbindung oxidiert,
- D): den Glucoserest in 8-Stellung der Anthrachinonverbindung in saurem Medium abspaltet und
- E): die erhaltene 1,8-Dihydroxyanthrachinonverbindung acetyliert und das Diacetylrhein gewinnt.

Die einzelnen Stufen des Verfahrens werden nachfolgend erlautert:

### Reduktion der Sennoside

Das als Ausgangsmaterial dienende Sennoridgemisch läßt sich beispielsweise aus der Sennadroge gewinnen.

Die Sennadroge besteht aus den getrockneten Blättern und Früchten der Sennespflanze, beispielsweise der indischen Senna (Cassia anguatifolia) und der ägyptischen Senna (Cassia acutifolia). Die Sennadroge enthält Dianthron-glucoside von Rhein und Aloe-Emodin. Die wichtigsten sind die Bennoside A, B, Al, C, D und D1. Die Sennoside entsprechen der Formel:

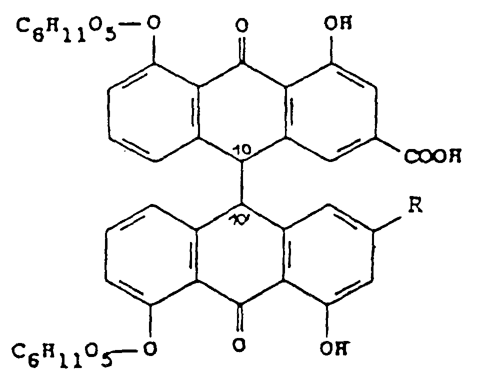

Bei den Sennosiden A, B und A1 steht R für COOH und bei den Sennosiden C, D und D1 steht R für CH₂OH. Die Sennoside A, B und A1 bzw. C, D und D1 sind Stereoisomere und unterscheiden sich untereinander durch die Konfiguration an den C-Atomen 10 und 10'.

Die Gewinnung der Sennoside aus der Sennadroge ist beispielsweise in der DE-A-32 00 131 beschrieben, auf die hiermit in vollem Umfang Bezug genommen wird. Danach extrahiert man zunächst die Sennadroge mit wäßrigem Methanol. Das nach vollständigem Entfernen des Methanols verbleibende Konzentrat enthält die Sennoside in Form des Kaliumsalzes. Dieses Konzentrat ist als Ausgangsmaterial für das erfindungsgemäße Verfahren geeignet. Man kann das Konzentrat noch durch Flüssig-Extraktion mit in Wasser teilweise löslichen Alkoholen oder Ketonen (z.B. Butanol-2, 2-Butanon) (Raffinat) reinigen. Das Raffinat wird auf einen pH-Wert von etwa 1,5 bis 2,0 angesäuert und die Sennoside werden unter Animpfen zur Kristallisation gebracht. Das erhaltene Rohsennosidgemisch ist ebenfalls als Ausgangsprodukt für das erfindungsgemäße Verfahren brauchbar. Gewünechtenfalls kann man das Rohsennosidgemisch auch noch umkristallisieren.

Alternativ kann man das mit einem in Wasser teilweise löslichen Alkohol oder Keton, insbesondere Butanol-2, versetzte Konzentrat als Ausgangsprodukt verwenden.

Bei der Extraktion der Sennadroge beträgt das Verhältnis von Droge zu Extraktlösungsmittel vorzugsweise 1 : 4 bis 1 : 15, insbesondere 1 : 4 bis 1 : 10.

Die Extraktion wird vorzugsweise in Gegenwart eines Puffers durchgeführt, beispielsweise Trinatriumcitrat, Glycin, Natriumbikarbonat oder Saccharose.

Nach den erfindungsgemäßen Verfahren werden diese Ausgangsmaterialien einer vollständigen Reduktion zu dem entsprechenden Rhein-9-anthron-8-glucosid (R=COOH) und dem entsprechenden Aloeemodin-9-anthron-8-glucosid (R=CH₂OH) der Formel: 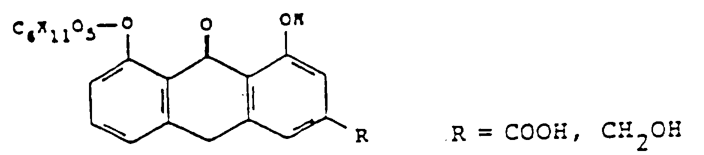 unterworfen.

Reduktionsmittel mit einem geeigneten Reduktionspotential sind z.B. Zinn-II-chlorid, Schwefeldioxid, Alkalimetallborwasserstoffe und vorzugsweise Alkalimetalldithionite, insbesondere Natriumdithionit.

Zur Durchführung der Reduktion kann man das Ausgangsmaterial in wäßriger Lösung oder Suspension vorlegen und das Reduktionsmittel in fester Form oder in Wasser gelöst zugeben. Man kann auch in einem 2-Phasengemisch arbeiten, indem man ein mit Wasser teilweise mischbares, polares organisches Lösungsmittel, insbesondere 2-Butanol oder Aceton, zugibt.

Man kann bei Umgebungstemperatur oder höherer Temperatur reduzieren. Die Reduktion wird zweckmäßigerweise bei 40-60 °C, insbesondere bei 50 - 55 °C durchgeführt. Man arbeitet bei schwach saurem bis schwach alkalischem pH-Wert der Ausgangssennosidlösung bzw. -Suspension, vorzugsweise bei pH 7 - 9. Gewünschtenfalls kann man die Reduktion mehrfach, insbesondere 2 bis 10 mal durchführen.

Die gebildeten 9-Anthron-8-glucoside fällt man durch Zugabe einer Säure, beispielsweise Schwefelsäure, bis pH 4 bis 4,5 aus. Die Temperatur sollte dabei zweckmäßigerweise nicht mehr als 40 °C betragen. Zweckmäßigerweise arbeitet man bei der Ausfällung der 9-Anthron-8-glucoside und bei ihrer Isolierung (beispielsweise durch Filtration) unter Stickstoff, um eine unkontrollierte Oxidation dieser Verbindung zu vermeiden.

Es ist wesentlich, daß die Reduktion vollständig verläuft. Zweckmäßigerweise verwendet man daher das Reduktionsmittel in großem Überschuß. Bei Verwendung von Natriumdithionit verwendet man im allgemeinen die 1- bis 4fache Gew.-Menge an Natriumdithionit, bezogen auf den Gehalt des Ausgangsmaterials an Sennosiden. Außerdem läßt man das Reduktionsmittel mindestens 2 h, vorzugsweise mindestens 3 h einwirken. Im allgemeinen erfolgt die Reduktion nicht länger als 10 h. Vorzugsweise führt man eine Nachreduktion unter den genannten Bedingungen durch.

Das erhaltene Produkt wird vor seinem Einsatz in der nächsten Stufe vorzugsweise umgefällt, indem man es in wäßriger Lösung durch Zugabe einer Base (NaOH, KOH) bis etwa pH-Wert 6 - 7 in Lösung bringt, die wäßrige Lösung mit 2-Butanol, Aceton oder 2-Butanon extrahiert und das Produkt durch Zugabe einer Säure bis etwa pH-Wert 2 - 4 wieder ausfällt.

### Flüssig-Flüssig-Verteilung

In dieser Stufe werden die Aloeemodinkomponenten, insbesondere das Aloeemodin-9-anthron-8-glucosid, entfernt. Hierfür wird eine Flüssig-Flüssig-Verteilung des erhaltenen Produktes in einem mit Wasser nur teilweise mischbaren polaren organischen Lösungsmittel und einer wäßrigen Phase vorgenommen. Geeignete polare organische Lösungsmittel sind C₄-C₅-Alkanole und Di-C₁-C₃-Alkylketone, wie Aceton, 1-Butanol, 2-Butanol und 2-Butanon. Vorzugsweise verwendet man 2-Butanol oder Aceton.

Vorzugsweise gibt man zu der wäßrigen Phase ein Reduktionsmittel, um der wäßrigen Phase während der gesamten Flüssig-Flüssig-Verteilung ein Redox-Potential von -210 mV oder negativer zu verleihen. Zweckmäßigerweise verwendet man das gleiche Reduktionsmittel wie in Stufe A. Bei Verwendung eines Alkalimetalldithionits als Reduktionsmittel ist im allgemeinen eine 2-4 Gew.-%ige Lösung bei einem pH-Wert von 7 bis 11 ausreichend, um die erwähnten Potentialbedingungen einzuhalten.

Das Volumenverhältnis von wäßriger Phase (schwere Phase) zu organischer Phase (leichte Phase) liegt im allgemeinen im Bereich von 1:5 bis 1:40.

Vorzugsweise erfolgt die Flüssig-Flüssig-Extraktion im Gegenstrom. Das Gemisch der Anthronverbindungen wird dabei in Form der nach der Reduktion erhaltenen Lösung oder, wenn die Anthronverbindungen isoliert wurden, in Form einer 3-15 Gew.-%igen Lösung zugeführt.

Nach der Verteilung befindet sich das gewünschte Rhein-9-anthron-8-glucosid in der wäßrigen Phase. Es wird durch Zugabe einer Säure bis zu einem pH-Wert von etwa 2 bis 4 gefällt und in üblicher Weise gewonnen.

### Oxidation des Rhein-9-anthron-8-glucosids

Das erhaltene Rhein-9-anthron-8-glucosid wird nun zu Rhein-8-glucosid der Formel: 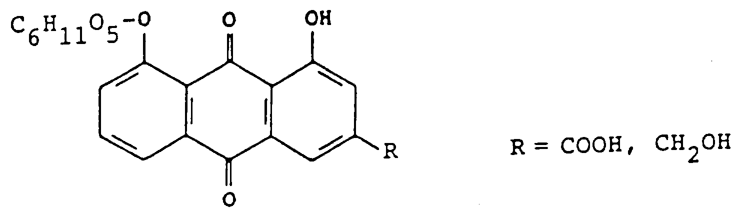 oxidiert. Geeignete Oxidationsmittel für diesen Zweck sind z.B. Sauerstoff, Peroxidverbindungen (Wasserstoffperoxid), Mangan-, Chrom-oder Eisenverbindungen in den hohen Oxidationsstufen. Vorzugsweise verwendet man ein Eisen-III-Salz, insbesondere Eisen-III-Sulfat. Zweckmäßigerweise arbeitet man bei erhöhter Temperatur, jedoch unterhalb 60 °C. Dadurch wird vermieden, daß unerwünschte und undefinierbare Oxidationsprodukte entstehen. Nach beendeter Oxidation wird das gebildete Rhein-8-glucosid in üblicher Weise isoliert.

### Abspaltung des Glucoserestes

Der Glucoserest in 8-Position wird in saurer Lösung abgespalten. Zweckmäßigerweise arbeitet man bei etwa 85 bis 95 °C. Das erhaltene Produkt wird in üblicher Weise isoliert.

Es ist bekannt, Sennoside nach saurer Hydrolyse durch Umsetzung mit Eisen-III-Chlorid direkt in Rhein zu überführen, siehe beispielsweise DE-A-27 11 493. Dabei beträgt die Ausbeute jedoch nur etwa 10 % und außerdem ist das gebildete Rhein schwer abzutrennen.

Beim erfindungsgemäßen Verfahren wird die reduktive Spaltung der Sennoside, die Oxidation der gebildeten Anthronverbindungen zu den entsprechenden Anthrachinon-Verbindungen und die Abspaltung des Glucoserestes in 8-Stellung der Anthrachinonverbindungen in jeweils getrennten Stufen durchgeführt. Im Anschluß an die reduktive Spaltung werden alle Verbindungen, die im weiteren Verlauf zur Bildung von Aloeemodin oder dessen Derivaten führen können, durch Flüssig-Flüssig-Verteilung quantitativ entfernt. Zudem ist es möglich, die Oxidation bei schonenden Temperaturen durchzuführen, so daß die Bildung von unerwünschten und undefinierbaren Oxidationsprodukten vermieden wird. Außerdem kann bei dieser Reaktionsführung das eingesetzte Eisensalz nahezu quantitativ wiedergewonnen und nach Rückoxidation erneut verwendet werden. Die Trennung von Oxidationsschritt und Hydrolyseschritt erlaubt es, aufgrund der höheren Wasserlöslichkeit der Anthronglucoside im Vergleich zu den betreffenden Aglyka, die Oxidation schonend bei Raumtemperatur unter 60 °C durchzuführen, wodurch die sonst unvermeidliche Bildung undefinierbarer Nebenprodukte vermieden wird.

### Acetylierung der 1,8-Dihydroxyanthrachinonverbindung

Die Acetylierung der erhaltenen 1,8-Dihydroxyanthrachinonverbindungen erfolgt in üblicher Weise. Beispielsweise kann man mit Acetanhydrid in Gegenwart von Natriumacetat acetylieren, wie in Arch. Pharm. 241, 607 (1903) beschrieben. Die Acetylierung kann jedoch auch mit anderen, dem Fachmann bekannten Methoden erfolgen, beispielsweise durch Umsetzung mit Acetylchlorid etc.

Das auf diese Weise erhaltene Diacetylrhein ist im wesentlichen frei von Aloeemodin und Derivaten davon. Der Gehalt an diesen Verunreinigungen beträgt dabei noch ca. 50 ppm (bestimmt nach den in den Beispielen beschriebenen Analyseverfahren). Der Gehalt an diesen Verunreinigungen kann weiter gesenkt werden, wenn man das erhaltene Diacetylrhein auf folgende Weise umkristallisiert:

Man überführt das Diacetylrhein in ein Alkalimetallsalz, indem man es mit einer geeigneten Base behandelt. Eine geeignete Base ist beispielsweise ein Alkalimetallacetat, vorzugsweisse Kaliumacetat. Man verwendet vorzugsweise äquimolare Mengen der Base und einen wäßrigen C₁-C₃-Alkohol, beispielsweise 80- bis 90%iges Ethanol, als Reaktionsmedium. Man läßt das Alkalimetallsalz des Diacetylrhein in der Kälte auskristallisieren, nimmt es in einem wäßrigen C₁-C₃-Alkohol auf und fällt es durch Zusatz einer Säure bis zu einem pH-Wert von etwa 3. Das ausgefallene Diacetylrhein wird dann in üblicher Weise isoliert und aufgearbeitet.

Das so erhaltene Produkt enthält weniger als 20 ppm der oben erwähnten Verunreinigungen. Außerdem liegt das Produkt in Form von nadelförmigen Kristallen vor, die für die galenische Formulierung besonders geeignet sind.

Das Produkt kann in üblicher Weise getrocknet werden. Zweckmäßigerweise wird man zunächst die Trocknung im Vakuum bei relativ niedriger Temperatur, beispielsweise nicht mehr als 40 °C so lange durchführen, bis der Wassergehalt des Produktes auf ca. 3 % oder weniger gefallen ist. Anschließend kann man die Temperatur auf 70 bis 110 °C erhöhen.

Die Erfindung betrifft auch das erfindungsgemäß erhältliche, im wesentlichen reine Diacetylrhein sowie ein pharmazeutisches Mittel, das diese Verbindung enthält. Die Anwendungsgebiete die zu verabreichende Dosis und geeignete Dosierungsformen sind bekannt, siehe US-A-4244968, 4346103, 4950687, DE-A-2711493 sowie Drugs Exptl. Clin. Res. 6 (1) 53 bis 64 (1980).

Die nachfolgenden Beispiele erläutern die Erfindung.

### Beispiel 1

Gewinnung des als Ausgangsmaterial verwendeten Sennosidgemisches:

Man gibt jeweils 40 kg Sennadroge (Sennosidgehalt ca. 1,5%) in zwei in Reihe geschaltete Perkolatoren mit einem Volumen von 250 l und bedeckt sie mit einer gelochten Stahlplatte. Als Lösungsmittel für die Extraktion verwendet man 70%iges Methanol, das auf die Droge im ersten Perkolator geleitet wird. Die im ersten Perkolator gebildete Lösung leitet man auf die Droge, die sich im zweiten Perkolator befindet. Dabei läßt man das Lösungsmittel frei durch den ersten Perkolator fließen.

Für die Extraktion von 40 kg Sennadroge verwendet man insgesamt 160 l Lösungsmittel. Nachdem man dieses Volumen 70%iges Methanol durch die beiden Perkolatoren geleitet und die entsprechende Menge Perkolat aufgefangen hat, koppelt man den Entleerungsschlauch des Perkolators mit einem Nachperkolatbehälter und leitet noch zusätzlich 60 l 70%iges Methanol durch die Perkolatoren. Danach leitet man das restliche freie Lösungsmittel aus dem ersten Perkolator in den oberen Teil des zweiten Perkolators und sammelt das Nachperkolat, bis es insgesamt 120 l ausmacht. Dann entleert man den ersten Perkolator, füllt ihn erneut mit 40 kg Sennadroge und pumpt das Nachperkolat auf die Droge, wobei 120 l Nachperkolat ausreichen, um die Droge im Perkolator zu bedecken. Anschließend bringt man die Temperatur der Lösung auf +30 °C.

Man verbindet diesen Perkolator mit dem zuvor extrahierten und führt die Extraktion wie oben beschrieben aus.

Für jeweils 40 kg Droge sammelt man 160 l Perkolat, aus dem man das Methanol in einem Vakuumrotationsverdampfer, der mit einer Füllkörpersäule ausgestattet ist, entfernt. Man erhält ca. 30 l Bodenprodukt. Dieses Konzentrat wird mit dem gleichen Volumen wassergesättigtem Butanol-2 extrahiert.

### Stufe A:

### Reduktion der Sennoside zu Rhein-9-anthron-8-glucosiden

1,0 l des extrahierten Konzentrats werden mit 48%iger Natronlauge auf pH-Wert 7,5 gebracht. Man erhitzt auf 60 °C und gibt unter Rühren während einer halben Stunde 90 g Natriumdithionit in fester Form in die Lösung. Nach beendeter Zugabe rührt man eine weitere Stunde. Anschließend gibt man unter Rühren konzentrierte Schwefelsäure bis zu einem pH-Wert von 2 zu. Man kühlt im Laufe von zwei Stunden auf Umgebungstemperatur, filtriert den ausgefallenen kristallinen Niederschlag ab und wäscht ihn mit schwefeldioxidhaltigem Wasser.

Gewünschtenfalls wird das rohe Rhein-9-anthron-8-glucosid umgefällt. Der noch feuchte Filterkuchen wird in einer Mischung von 15 Vol.-Teilen 2-Butanol und 85 Vol.-Teilen Wasser, die 0,5 Gew.-% Natriumpyrosulfit enthält, so gelöst, daß man durch Zugabe von 48%iger Natriumhydroxidlösung bis pH-Wert 7 eine 10%ige Lösung (G/V) erhält. Die Lösung wird mit konzentrierter Salzsäure auf auf pH-Wert 2,8 oder darunter angesäuert und während 2 h stehengelassen. Der ausgefallene Niederschlag wird abfiltriert und mit schwefeldioxid- oder natriumpyrosulfithaltigem Wasser gewaschen und getrocknet.

### Ausbeute: 90 %.

Mit dem auf diese Weise erhaltenen Produkt führt man eine erneute Reduktion (Nachreduktion) wie folgt durch:

3,0 g des rohen getrockneten Rhein-9-anthron-8-glucosids oder die entsprechende Menge des feuchten Produktes löst man zusammen mit 1,4 g Natriumdithionit und 2,3 ml 5N NaOH in 15 ml Wasser. Anschließend füllt man mit Wasser auf 24 ml auf und erwärmt die Lösung 20 Minuten bei 55 °C. Danach gibt man weitere 1,5 g Natriumdithionit in die Lösung und erwärmt 20 Minuten bei 55 °C. Anschließend gibt man 0,9 ml 5N NaOH und 1,5 g Natriumdithionit zu. Nach 20 minütigem Erwärmen auf 55 °C gibt man noch einmal 0,9 ml 5N NaOH. Die erhaltene Lösung wird direkt in die nachfolgende Flüssig-Flüssig-Extraktion eingeführt.

### Stufe B:

### Abtrennung der Aloeemodin-Komponenten

Die Abtrennung der Aloeemodin-Komponenten erfolgt durch Flüssig-Flüssig-Verteilung der 9-Anthron-8-glucoside im Gegenstrom mit Hilfe einer Apparatur aus 60 Mischer-Scheider-Einheiten (Mixer-Settler-Apparatur). Als wäßrige, schwerere Phase verwendet man eine Lösung von 3,0 g Natriumdithionit in 3,5 ml 5N NaOH und 96 ml Wasser. Als organische, leichtere Phase verwendet man (wassergesättigtes) 2-Butanol oder Aceton. Die beiden Phasen werden so durch die Apparatur geschickt, daß das Volumenverhältnis von schwerer Phase zu leichter Phase 1:10 beträgt.

Das aufzutrennende Gemisch wird in Form der frisch reduzierten Lösung oder in Form einer Lösung von entsprechendem pH-Wert und von entsprechender Konzentration, welche die aus Stufe A erhaltenen 9-Anthron-8-glucoside enthalten, der Apparatur zugeführt, und zwar derart, daß pro Volumenteil des aufzutrennenden Gemisches 30 Vol.-Teile der organischen Phase verwendet werden.

Der pH-Wert der das Gemisch enthaltenden Lösung wird mit Hilfe eines Glycinpuffers bei 9 - 9,5 gehalten. Der Puffer aus 3 Vol.-Teile einer 7,5%igen Glycinlösung 1 Vol.-Teil 1N NaOH wird in einer Menge von 240 ml Pufferlösung pro 150 g rohes Rhein-9-anthron-8-glucosid zugegeben. Die unerwünschten Aloeemodinverbindungen reichern sich in der organischen Phase an, während das Rhein-9-anthron-8-glucosid in der wäßrigen Phase verbleibt. Die wäßrige Phase wird mit Schwefelsäure bis pH-Wert 2,8 angesäuert, der gebildete Niederschlag wird abfiltriert und mit Wasser und Aceton gewaschen und an der Luft bei Umgebungstemperatur getrocknet. Man erhält auf diese Weise Rhein-9-anthron-8-glucosid mit einem Gehalt an Aloeemodinkomponenten von 41 ppm bestimmt als Aloeemodin nach einer Methode, die am Ende dieser Patentanmeldung beschrieben wird.

Ausbeute 97 %, bezogen auf Rhein-9-anthron-8-glucosid.

### Stufe C:

### Oxidation zu Rhein-8-glucosid

Das Produkt aus Stufe B (bezogen auf einen Gehalt von 3,0 kg Sennoside A, A₁ und B) wird in einer Lösung aus 184 l entmineralisiertem Wasser und 75,5 kg Eisen-III-sulfathydrat (22% Fe³⁺) suspendiert. Die Suspension wird auf 55-62 °C erhitzt und 14 h unter Anwendung eines schnell laufenden Dispergators oxidiert. Ist die Oxidation beendet, wird das gebildete Rhein-8-glucosid abfiltriert und mit 50 l entmineralisiertem Wasser, das mit Schwefelsäure auf pH 2 eingestellt worden ist, gewaschen.

### Stufe D:

### Hydrolyse zu Rhein

Der feuchte Filterrückstand aus Stufe C wird in 200 kg 20 Gew.-%iger Schwefelsäure suspendiert und 8 h bei 88-92 °C gerührt. Das gebildete Rhein wird abfiltriert und kann zur Lagerung bei 1 mbar Vakuum 48 h bei 40 °C getrocknet oder auch im feuchten Zustand sofort zur Acetylierung in Stufe E eingesetzt werden.

Gesamtausbeute für die Stufen A bis D: 79%, bezogen auf in Stufe A eingesetzte Sennoside A, A₁ und B.

### Stufe E:

### Acetylierung zu Diacetylrhein

6,5 kg Rhein aus Stufe D werden in 100 1 Essigsäureanhydrid 10 Min. suspendiert, mit 2 kg Kaliumacetat versetzt, auf 95 °C unter Rühren erwärmt, mit 0,65 kg Aktivkohle versetzt und 1/2 h bei 90 - 95°C gerührt. Die Aktivkohle wird aus der heißen Lösung herausfiltriert und das Filtrat bei 90 °C mit 2,1 kg 96-98 Gew.-%iger Schwefelsäure versetzt. Anschließend wird unter Rühren möglichst schnell auf 20 °C gekühlt. Die entstandene Suspension wird filtriert. Der Rückstand wird mit entmineralisiertem Wasser sulfatfrei gewaschen.

Ausbeute: 83 %.

### Stufe F:

### Umkristallisation, Trocknung, Vermahlung

Unter schnellem Rühren werden 7,5 kg Diacetylrhein aus Stufe E (bezogen auf getrocknete Substanz) in 375 1 90 Vol.-prozentigem Ethanol suspendiert. Die Suspension wird auf 70 °C erhitzt und dann mit 3,75 kg Kaliumacetat versetzt. Beim Abkühlen auf 0-2 °C kristallisiert aus der zwischendurch entstandenen, klaren Lösung das reine Kaliumsalz des Diacetylrheins aus.

Das Kaliumsalz wird abfiltriert und in 300 l 40 Vol.-prozentigem Ethanol unter Zusatz von 3 kg Kaliumacetat bei 20-30 °C gelöst. Die klare Lösung wird mit 10 Gew.-prozentiger Schwefelsäure auf pH-Wert 3,0 eingestellt. Das auskristallisierte Diacetylrhein wird abfiltriert und mit demineralisiertem Wasser sulfatfrei gewaschen.

Die Trocknung des Produkts erfolgt zunächst im Vakuum bei 1 mbar und 40 °C innerhalb 24 Std. Ist der Restwassergehalt unter 3 % gefallen, wird das Material grob zerkleinert und bei 1 mbar Vakuum und 7C °C 24 Std. nachgetrocknet.

Anschließend wird bei einer Siebeinlage von 0,5 mm vermahlen und zur Entfernung von Lösungsmittelrückständen bei 1 mbar Vakuum und 70 °C nachgetrocknet.

Ausbeute 95 %

### Beispiel 2

Man wiederholt die im Beispiel 1 beschriebene Extraktion der Sennadroge und die Reduktion der Sennoside. Die Nachreduktion führt man dann wie folgt durch:
Man löst 14,0 g Saccharose, 4,5 g Natriumdithionit (85 %) und 13,3 g Kaliumacetat in 133 ml Wasser und gibt 1,3 ml 48%ige Natriumhydroxidlösung und 17,3 g Kaliumcarbonat zu. Anschließend versetzt man mit 293 ml Aceton und 50 ml Wasser. Man schüttet die Mischung in einen Scheidetrichter und trennt die Phasen, wobei man 375 ml obere Phase (Acetonphase) und 130 ml untere Phase erhält.

In 98 ml der unteren Phase löst man 1,4 ml 48%ige Natriumhydroxidlösung und 10 g rohes Rhein-9-anthron-8-glucosid. Man erwärmt auf 45 bis 50 °C und hält 20 bis 30 Minuten bei dieser Temperatur. Anschließend gibt man 1,0 ml 48%ige Natriumhydroxidlösung und 3,4 g Natriumdithionit zu und erwärmt weitere 20 - 30 Min. auf 45 - 50 °C. Anschließend gibt man erneut 1,0 ml 48%ige Natriumhydroxidlösung und 3,4 g Natriumdithionit zu und erwärmt 20 - 30 Min. auf 45 - 50 °C.

Die Abtrennung der Aloeemodin-Komponente erfolgt durch Flüssig-Flüssig-verteilung der reduzierten Lösung im Gegenstrom gegen die oben erwähnte obere Phase (Acetonphase). Die abfließende, das Rhein-9-anthron-8-glucosid enthaltende Raffinatphase wird auf 400 ml verdunstet und mit 20 ml Butanol-2 versetzt. Man gibt Salzsäure oder Schwefelsäure bis pH-Wert 4,0 bis 4,2 zu. Der gebildete Niederschlag wird abfiltriert, mit 40 ml Wasser und 30 ml Aceton gewaschen und anschließend getrocknet. Die anschließende Oxidation erfolgt wie im Beispiel 1 beschrieben.

### Beispiel 3

Das nach der Extraktion der Sennadroge erhaltene Konzentrat wird mit ca. 2 l Butanol-2 versetzt. Die Reduktion des Sennesfrüchtekonzentrat/Butanol-2-Gemisches wird dann in 7 Stufen unter Stickstoff als Schutzgas durchgeführt. Nach der Reduktionsstufe I erfolgt eine Fällung des rohen Rhein-9-anthron-8-glucosids.

### Reduktionsstufe I

100 l Sennesfrüchtekonzentrat/Butanol-2-Gemisch, enthaltend ca. 4 kg Sennoside, werden in einem Rührbehälter vorgelegt und mit Stickstoff überlagert. Unter Rühren werden 6 l 20%ige (w) Natronlauge, danach 350 l wassergesättigtes Butanol-2 (z.B. aus Stufe II) nacheinander zugesetzt und 15 Min. gerührt. Der Ansatz wird auf 42 - 50 °C aufgeheizt und mit 7 kg Natriumdithionit versetzt. Es wird noch 45 Min. gerührt. Der pH-Wert wird mit 20%iger (w) Natronlauge bei 7,5 - 8 gehalten. Das Reduktionspotential (gegen Ag/AgCl-Elektrode) wird erforderlichenfalls durch Natriumdithionitzugabe auf unter -630 mV gehalten. Nach Kühlung auf 30 - 35 °C wird mit 10%iger (w) Schwefelsäure bis pH < 4 innerhalb 1,5 Std. gefällt. Die entstehende Suspension wird bei kleiner Rührgeschwindigkeit < 25 °C ca. 10 Std. gerührt. Der entstandene Niederschlag wird abfiltriert. Der Niederschlag wird in 60 l 15%igem (w) Butanol-2 suspendiert, 30 Min. bei 50 - 60 °C gerührt und anschließend filtriert. Der Rückstand wird mit 100 l demineralisiertem Wasser gewaschen. Die Rohausbeute an Rhein-9-anthron-8-glucosid, bezogen auf eingesetzte Sennoside, liegt über 82 %.

### Reduktionsstufe II

3,3 kg rohes Rhein-9-anthron-8-glucosid aus Stufe I werden in einer Mischung aus 42 l demineralisiertem Wasser und 7,4 1 Butanol-2 suspendiert. Mit 2 l 20%iger (w) Natronlauge und 9,9 kg Trinatriumcitrat wird die Suspension in Lösung gebracht und danach mit 3,3 kg Natriumdithionit und 350 l wassergesättigtem Butanol-2 (z.B. aus Stufe III) versetzt. Der Ansatz wird auf 42 - 45 °C aufgeheizt. Der pH-Wert wird mit 20%iger (w) Natronlauge bei 8,5 - 9 gehalten. Das Reduktionspotential (gegen Ag/AgCl-Elektrode) wird erforderlichenfalls durch Natriumdithionit-Zugabe auf unter -750 mV gehalten.

Nach einer Standzeit von 30 Min. wird die Oberphase entnommen und die Unterphase in der Stufe III weiterverarbeitet.

### Reduktionsstufe III

Mit der Unterphase aus der Stufe II wird unter Zusatz der folgenden Chemikalien das in Stufe II beschriebene Reduktions-/Extraktionsverfahren wiederholt:

| | |
|---|---|
| 1,65 kg | Natriumdithionit |
| 0,8 l | 20%ige (w) Natronlauge |
| 350 l | wassergesättigtes Butanol-2 (z.B. aus Stufe IV) |

### Reduktionsstufen IV - VII

Mit der Unterphase aus der jeweils vorausgegangenen Stufe wird unter Zusatz der folgenden Chemikalien das in Stufe II beschriebene Reduktions-/Extraktionsverfahren wiederholt:

| | |
|---|---|
| 0,825 kg | Natriumdithionit |
| 0,4 l | 20%ige (w) Natronlauge |
| 350 l | wassergesättigtes Butanol-2 (z.B. aus dem jeweils nachfolgenden Stufen - Gegenstromprinzip) |

Die in der Stufe VII abgetrennte Unterphase wird auf 30-35 °C gekühlt und das Rhein-9-anthron-8-glucosid - wie in Stufe I beschrieben - ausgefällt. Der entstandene Niederschlag wird abfiltriert und mit 100 l entmineralisiertem Wasser gewaschen. Anschließend wird mit 10 l Eisen-III-sulfatlösung (Herstellung siehe Stufe B, Beispiel 1) überdeckt.

Das Rhein-9-anthron-8-glucosid wird dann, wie im Beispiel 1 oder 2 beschrieben, in die Sennoside überführt.

### Pharmakologische Untersuchungen

Die Wirksamkeit von Diacetylrhein wurde bei chronischen Entzündungsmodellen nach oraler Verabreichung bestimmt. Folgende Versuchsmodelle kamen zur Anwendung:
Cotton-Pellet-Granulom bei der Ratte und durch intraartikuläre Applikation von Vitamin A ausgelöste Arthrose beim Kaninchen.

a) Cotton-Pellet-Granulom bei der Ratte.
   Junge, geschlechtsreife Ratten (n - 10) erhielten 25, 50 oder 100 mg Diacetylrhein/kg bzw. 5 mg Indomethacin/kg oder 100 mg Acetylsalicylsäure/kg täglich für 5 Tage. Eine nur mit Wasser behandelte Kontrollgruppe wurde ebenfalls mitgeführt. Die Implantation der Pellets erfolgte am ersten Behandlungstag. Frisch- und Trockengewichte der am Versuchsende präparierten Granulome zeigten eine signifikante und deutliche dosisabhängige Verringerung im Vergleich zur Kontrollgruppe. Dabei entsprach die Wirkung von 100 mg Diacetylrhein/kg in etwa der Wirkung von 5 mg Indomethacin oder 100 mg Acetylsalicylsäure. Die Thymus- und Nebennierengewichte änderten sich während der Behandlung nicht.
b) Vitamin-A-Arthrose.
   Durch drei intraartikuläre Injektionen von 30 000 IE Vitamin A während 9 Tagen wurde in zwei Gruppen von je 10 Kaninchen (weiße Neuseeländer) eine arthroseähnliche Gelenkveränderung ausgelöst. 56 Tage später wurden 10 Tiere mit 3 mg Diacetylrhein/kg/Tag für 8 Wochen behandelt. Im Vergleich zur Kontrollgruppe waren die makroskopisch und mikroskopisch erkennbaren Gelenkveränderungen in der Behandlungsgruppe signifikant verringert.
   Die kurative Wirkung von Diacetylrhein wurde weiterhin mit der von Acetylsalicylsäure an je 7 Kaninchen verglichen, die nach 6-tägiger Vorbehandlung mit dreimal 10 000 IE Vitamin A und einem 26-tägigen behandlungsfreien Intervall für 8 Wochen entweder 5 mg Diacetylrhein/kg/Tag (Versuchsgruppe), 15 mg Acetylsalicylsäure/kg/Tag (positive Kontrollgruppe) erhielten oder unbehandelt blieben (negative Kontrollgruppe). In allen drei Gruppen traten 24 Tage nach der letzten Vitamin A-Injektion vergleichbare Bewegungsstörungen in Form von nachziehenden Hinterläufen auf. In der negativen Kontrollgruppe verstärkten sich während der folgenden 8 Wochen die klinischen Zeichen einer manifesten Arthose.
   In der Versuchsgruppe und der positiven Kontrollgruppe besserten sich diese Symptome unter der 8-wöchigen Behandlung signifikant.

### Magenschleimhautveränderungen

Während die einmalige Gabe von 400 mg Diacetylrhein/kg oder des Lösungsmittels bei der Ratte keine Erosionen der Magenschleimhaut verursachte, fanden sich nach Gabe von Ibuprofen (200 mg/kg) oder Indomethacin (20 mg/kg) eindeutige Schleimhautschäden von punktförmigen (1 mm Durchmesser) bis großen (3 mm Durchmesser) Erosionen. Auch die zweimal tägliche Gabe von 100 mg Diacetylrhein/kg über 3 Tage löste keine Schleimhautschäden aus, wohl aber die entsprechende Anwendung von 10 mg Indomethacin/kg. Dabei handelte es sich um Erosionen von 1 - 3 mm Durchmesser.

### Toxikologie

Die akute Toxizität LD₅₀ beträgt je nach untersuchter Spezies (Ratte, Maus, Katze) nach oraler Anwendung 1,9 bis 7,9 g/kg. Dabei erwies sich die Ratte als am wenigsten empfindlich. Nach parenteraler Gabe (i.v. i.p.) lagen die LD₅₀-Werte bei diesen Spezies zwischen 119 und 339 mg/kg.

### Klinische Untersuchungen

1. In einer Doppelblind-Studie gegen Naproxen und anschließender Plazebo-Nachbehandlung wurde die Wirkung von Diacetylrhein bei Coxarthrose und Gonarthrose bei 95 (49/46) Patienten untersucht. Die applizierte Dosis war 50 mg Diacetylrhein-2 mal täglich bzw. 750 mg Naproxen täglich. Die Behandlungsdauer war 60 Tage nach 7-tägiger wash-out-Phase. Die anschließende Plazebo-Behandlung erstreckte sich über 60 Tage.
   Prüfgrößen waren die Schmerz- und Beweglichkeits-Symptomatik nach einer Score-Scala, Funktionseinschränkung und Verträglichkeit.
   In beiden Behandlungsgruppen (Diacetylrhein/Naproxen) wurde hinsichtlich aller Prüfparameter eine statistisch signifikante Besserungsrate (P<0,01 bzw. P<0,05) im Vergleich zu den Ausgangswerten festgestellt. Nach Absetzen der Behandlung und anschließender Plazebogabe zeigte sich jedoch für die Diacetylrhein/Plazebo-Gruppe an den Tagen 90 und 120 hinsichtlich der Parameter Spontanschmerz, aktiver und passiver Bewegungsschmerz, eine statistisch signifikante Überlegenheit (P<0,01) im Vergleich zum Naproxen-/-Plazebo-Kollektiv. Dieser Unterschied wurde auf dem 5%-Niveau auch für den variablen Nachtschmerz und Druckschmerz 30 Tage nach Absetzen von Diacetylrhein gesichert.
2. In einer offenen Verlaufsstudie mit Kontrolle wurde die Wirkung von Diacetylrhein gegen Osteoarthrose der Wirbelsäule und des Knies bei 70 Patienten (35/35) untersucht. Die applizierte Dosis war 100 mg Diacetylrhein pro Tag. Die Behandlungsdauer betrug 60 Tage, die Beobachtungsdauer 75 Tage. Die Prüfgrößen waren Schmerz- und Bewegungseinschränkung. Die Größen wurden nach einem Score-System ermittelt.
   Die Kontrollgruppe umfaßte 35 Patienten, bei denen ausschließlich physio-therapeutische Maßnahmen durchgeführt wurden. In der Diacetylrhein-Behandlungsgruppe wurde ebenfalls eine Physiotherapie durchgeführt.
   Die Auswertung der Ergebnisse zeigte bezüglich aller Parameter eine statistisch signifikante Überlegenheit der Behandlungsgruppe gegenüber der Kontrollgruppe. Auch nach Absetzen der Behandlung konnte für die Diacetylrhein-Gruppe ein anhaltender therapeutischer Effekt ("hang-over-Effekt) festgestellt werden.
3. In einer single-blind, cross-over-Studie gegen Naproxen wurde die Wirkung von Diacetylrhein bei lokalisierter Arthrose bei 20 Patienten untersucht. Diese wurden in zwei Gruppen eingeteilt, wobei in der ersten Gruppe zunächst 2 mal 50 mg Diacetylrhein 20 Tage lang verabreicht wurde. Anechließend erfolgte drei Tage lang eine wash-out-Phase und eine weitere Behandlung mit 2 mal 250 mg Naproxen pro Tag an weiteren 20 Tagen. In der zweiten Gruppe wurde die umgekehrte Reihenfolge eingehalten. Die Behandlungsdauer betrug insgesamt 43 Tage. Prüfgrößen waren Schmerz, Kompressioneechmerz, passiver Bewegungsschmerz, Funktionseinschränkung und Schwellung nach einem Score-System.
   Die Auswertung der Ergebnisse zeigt eine Überlegenheit der Behandlung mit Diacetylrhein im Vergleich zur Behandlung mit Naproxen. Es wurden keine nennenswerten Nebenwirkungen beobachtet, auch keine Veränderungen der klinischen Laborparameter.
4. In einer randomisierten Doppelblindstudie in "double dummy-Technik" gegen Naproxen wurde die Wirkung von Diacetylrhein bei 23 Patienten (12/11) mit Osteoarthrose untersucht (Verträglichkeitsstudie). Die applizierte Dosis war 2 mal 50 mg Diacetylrhein pro Tag und 3 mal 250 mg Naproxen pro Tag. Die Behandlungsdauer betrug 4 Wochen. Die Prüfgrößen waren ösophagogastroduodenoskopische Befunderhebung vor und nach Therapie. Es wurden nur Patienten mit normalen Schleimhautbefunden bzw. mit leichten Schleimhautläsionen (Grad 1) in die Studie aufgenommen.
   Nach 4 Wochen zeigten die endoskopischen Befunde in einem Fall (10 %) in der Diacetylrheingruppe Schleimhautläsionen des Grades 2, während in der Naproxen-Behandlungsgruppe 5 Patienten (50 %) Schleimhautläsionen der Grade 2, 3 und 4 aufwiesen. In allen Fällen lag ein normaler Aufnahmebefund vor.

### Analytische Bestimmung von Aloeemodin

Man löst 50 mg Diacetylrhein in 25,3 ml 0,5 M NaOH in einem Scheidetrichter und schüttelt 10 Minuten. Anschließend gibt man 74,6 ml einer Lösung zu, die 0,5 M Glycin und 0,5 M NaCl enthält. Dabei ergibt sich ein pH von 9,5.

Diese Lösung extrahiert man 3 mal mit 25 ml Chloroform. Die vereinigten organischen Phasen werden 1 mal mit 10 ml 0,5 M eines Puffers von pH 9,5 (Glycin, NaOH, NaCl) und 1 mal mit 10 ml 0,01 M Schwefelsäure extrahiert. Man entfernt das Lösungsmittel der organischen Phase und löst den Rückstand in 1 ml Methanol.

Für die Standardlösung löst man 2 mg Aloeemodin in 20 ml N, N-Dimethylacetamid und verdünnt mit Methanol bis zu einer Konzentration von 2 µg/ml (entsprechend 40 ppm).

Der Gehalt der Lösungen wird mittels HPLC untersucht. Die Linearität der HPLC-Methode wurde mit Aloeemodin-Standardlösung im Bereich von 0,11 µg/ml (entsprechend 2,2 ppm) bis 53,6 µg/ml (entsprechend 1072 ppm) nachgewiesen. Die Gehaltsbestimmung erfolgt mit einer Merck HPLC-Säule Lichrocart 250-4, gepackt mit Li-Chrospher-100 RP-18, 5µm, bei 40 °C mit einer mobilen Phase aus 1%iger Essigsäure in Methanol (V/V), 1%iger Essigsäure in Wasser (V/V) und Acetonitril im Verhältnis von 49:46:5).

### Analytische Bestimmung des Produkts der Stufe B, und zwar Rhein-9-anthron-8-glucosid mit einem Gehalt an Aloeemodin-komponenten von 41 ppm, bestimmt als Aloeemodin

Die zu untersuchende Substanz wird durch Oxidation mit Eisen-III-chlorid unter gleichzeitiger Hydrolyse mit Salzsäure in einem 2-Phasengemisch aus wäßriger Lösung und Tetrachlorkohlenstoff in Rhein und Aloeemodin überführt. Das Rhein wird in ein Salz überführt, so daß es durch Flüssig-Flüssig-Verteilung von Aloeemodin abgetrennt werden kann. Das in der organischen Phase befindliche Aloeemodin wird per HPLC bestimmt.

## Patentansprüche

1. Verfahren zur Gewinnung von Diacetylrhein, das im wesentlichen frei von Aloeemodinkomponenten ist,
**dadurch gekennzeichnet**, daß man
A) Aloeemodinkomponente enthaltendes Rhein-9-anthron-8- glucosid einer Flüssig-Flüssig-Verteilung zwischen einem nur teilweise mit Wasser mischbaren, polaren organischen Lösungsmittel und einer wäßrigen Phase unterwirft,
B) die nach der Verteilung in der wäßrigen Phase enthaltenen Rhein-9-anthron-8-glucoside zu Rhein-8-glucosid oxidiert,
C) den Glucoserest in 8-Stellung des Rhein-8-glucosids in saurem Medium abspaltet und
D) das erhaltene Rhein acetyliert und das Diacetylrhein gewinnt.

2. Verfahren zur Herstellung von Diacetylrhein, das im wesentlichen frei von Aloeemodinkomponenten ist,
**dadurch gekennzeichnet,** daß man
A) ein Sennosidgemisch einer Reduktion zu den entsprechenden Rhein-9-anthron-8-glucosid- und Aloeemodinanthron-8-glucosidverbindungen unterwirft,
B) eine Flüssig-Flüssig-Verteilung der erhaltenen Verbindungen zwischen einem nur teilweise mit Wasser mischbaren polaren organischen Lösungsmittel und einer wäßrigen Phase durchführt,
C) die nach der Verteilung in der wäßrigen Phase enthaltenen Rhein-9-anthron-8-glucosidverbindungen zu der entsprechenden Anthrachinonverbindung oxidiert,
D) den Glucoserest in 8-Stellung der Anthrachinonverbindung in saurem Medium abspaltet und
E) die erhaltene 1,8-Dihydroxyanthrachinonverbindung acetyliert und das Diacetylrhein gewinnt.

3. Verfahren nach Anspruch 2,
**dadurch gekennzeichnet**, daß man in Stufe A als Reduktionsmittel ein Alkalimetalldithionit verwendet.

4. Verfahren nach Anspruch 3,
**dadurch gekennzeichnet,** daß man bei einem pH-Wert von 7 bis 9 arbeitet.

5. Verfahren nach einem der Ansprüche 2 bis 4,
**dadurch gekennzeichnet,** daß man die Reduktion mehrfach durchführt.

6. Verfahren nach einem der vorhergehenden Ansprüche,
**dadurch gekennzeichnet,** daß man als polares organisches Lösungsmittel Aceton oder 2-Butanol für die Flüssig-Flüssig-Verteilung verwendet.

7. Verfahren nach einem der vorhergehenden Ansprüche,
**dadurch gekennzeichnet,** daß man für die Flüssig-Flüssig-Verteilung eine wäßrige Phase verwendet, deren Redoxpotential -210 mV oder negativer ist.

8. Verfahren nach einem der vorhergehenden Ansprüche,
**dadurch gekennzeichnet**, daß man die Flüssig-Flüssig-Verteilung im Gegenstrom durchführt.

9. Verfahren nach einem der vorhergehenden Ansprüche,
**dadurch gekennzeichnet,** daß man als Oxidationsmittel ein Eisen-III-Salz, vorzugsweise Eisen-III-Sulfat, verwendet.

10. Verfahren nach Anspruch 2,
**dadurch gekennzeichnet,** daß das Sennosidgemisch erhältlich ist durch Extraktion der Sennadroge mit wäßrigem Methanol, vorzugsweise in Gegenwart eines Puffers.

11. Verfahren nach einem der vorhergehenden Ansprüche,
**dadurch gekennzeichnet**, daß man das erhaltene Diacetylrhein umkristallisiert, indem man das Diacetylrhein in ein Alkalimetallsalz überführt, dieses in einem wäßrigen C₁-C₃-Alkohol aufnimmt und das Diacetylrhein durch Zugabe einer Säure wieder ausfällt.

## Claims

1. Method for obtaining diacetylrhein which is substantially free from aloe-emodin components,
characterised in that
A) rhein-9-anthron-8-glucoside containing aloe-emodin components is subjected to a liquid-liquid partition between a polar organic solvent which is only partly miscible with water and an aqueous phase,
B) the rhein-9-anthron-8-glucoside contained in the aqueous phase after the partition is oxidised to rhein-8-glucoside,
C) the glucose radical in the 8-position of the rhein-9-glucoside is split off in an acid medium and
D) the rhein obtained is acetylated and the diacetylrhein is recovered.

2. Method for the preparation of diacetylrhein which is substantially free from aloe-emodin components,
characterised in that
A) a mixture of sennosides is reduced to the corresponding rhein-9-anthron-8-glucoside compounds and aloe-emodin-9-anthron-8-glucoside compounds,
B) a liquid-liquid partition of the compounds obtained is carried out between a polar organic solvent which is only partly miscible with water and an aqueous phase,
C) the rhein-9-anthron-8-glucoside compounds contained in the aqueous phase after the partition is oxidised to the corresponding anthraquinone compound,
D) the glucose radical in the 8-position of the anthraquinone compound is split off in an acid medium and
E) the 1,8-dihydroxyanthraquinone compound obtained is acetylated and the diacetylrhein is recovered.

3. Method according to claim 2, characterised in that an alkali metal dithionite is used as reducing agent in Step A.

4. Method according to claim 3, characterised in that the procedure is carried out at a pH value of from 7 to 9.

5. Method according to any one of claims 2 to 4, characterised in that the reduction is carried out several times.

6. Method according to any one of the preceding claims, characterised in that acetone or 2-butanone is used as polar organic solvent for the liquid-liquid partition.

7. Method according to any one of the preceding claims, characterised in that an aqueous phase the redox potential whereof is -210 mV, or more negative, is used for the liquid-liquid partition.

8. Method according to any one of the preceding claims, characterised in that the liquid-liquid partition is carried out in countercurrent.

9. Method according to any one of the preceding claims, characterised in that an iron(III) salt, preferably iron(III) sulphate, is used as oxidising agent.

10. Method according to claim 2, characterised in that the mixture of sennosides is obtainable by extraction of the senna drug with aqueous methanol, preferably in the presence of a buffer.

11. Method according to any one of the preceding claims, characterised in that the diacetylrhein obtained is recrystallised by converting the said diacetylrhein into an alkali metal salt, by taking this up in an aqueous C₁ - C₃ alcohol and again precipitating out the diacetylrhein through the addition of an acid.

## Revendications

1. Procédé de préparation de diacétylrhéine pratiquement exempte de constituants de type aloémodine, caractérisé en ce que
A) on soumet un rhéine-9-anthrone-8-glucoside contenant des constituants de type aloémodine à un partage liquide-liquide entre un solvant organique polaire qui n'est que partiellement miscible à l'eau et une phase aqueuse,
B) on oxyde en rhéine-8-glucoside le rhéine-9-anthrone-8-glucoside contenu dans la phase aqueuse après le partage,
C) on élimine en milieu acide le reste de glucose en position 8 du rhéine-8-glucoside, et
D) on acétyle la rhéine obtenue et on obtient la diacétylrhéine.

2. Procédé de préparation de diacétylrhéine pratiquement exempte de constituants de type aloémodine, caractérisé en ce que
A) on soumet un mélange de sennosides à une réduction pour former les composés rhéine-9-anthrone-8-glucoside et aloémodine-anthrone-8-glucoside correspondants,
B) on effectue un partage liquide-liquide des composés obtenus entre un solvant organique polaire qui n'est que partiellement miscible à l'eau et une phase aqueuse,
C) on oxyde les composés de rhéine-9-anthrone-8-glucoside contenus dans la phase aqueuse après le partage pour former le composé anthraquinonique correspondant,
D) on élimine en milieu acide le reste de glucose en position 8 du composé anthraquinonique, et
E) on acétyle le composé 1,8-dihydroxyanthraquinonique obtenu et on obtient la diacétylrhéine.

3. Procédé selon la revendication 2, caractérisé en ce que, dans l'étape A, on utilise un dithionite de métal alcalin comme agent réducteur.

4. Procédé selon la revendication 3, caractérisé en ce que l'on travaille à un pH compris entre 7 et 9.

5. Procédé selon l'une des revendications 2 à 4, caractérisé en ce que l'on effectue plusieurs fois la réduction.

6. Procédé selon l'une des revendications précédentes, caractérisé en ce que l'on utilise de l'acétone ou du 2-butanol comme solvant organique polaire pour le partage liquide-liquide.

7. Procédé selon l'une des revendications précédentes, caractérisé en ce que l'on utilise pour le partage liquide-liquide une phase aqueuse dont le potentiel redox est de -210 mV ou plus négatif.

8. Procédé selon l'une des revendications précédentes, caractérisé en ce que l'on effectue le partage liquide-liquide à contre-courant.

9. Procédé selon l'une des revendications précédentes, caractérisé en ce que l'on utilise comme agent d'oxydation un sel de fer III, de préférence le sulfate de fer III.

10. Procédé selon la revendication 2, caractérisé en ce que l'on peut obtenir le mélange de sennosides par extraction de la drogue de séné avec du méthanol aqueux, de préférence en présence d'un tampon.

11. Procédé selon l'une des revendications précédentes, caractérisé en ce que l'on recristallise la diacétylrhéine obtenue en transformant la diacétylrhéine en un sel de métal alcalin que l'on reprend dans un alcool en C₁-C₃ aqueux et en reprécipitant la diacétylrhéine par addition d'un acide.
